# EUROPÄISCHE PATENTSCHRIFT

(11) **EP 1 525 861 B1**
(45) Veröffentlichungstag und Bekanntmachung des Hinweises auf die Patenterteilung: **15.08.2007**
(21) Anmeldenummer: 04027208.0
(22) Anmeldetag: 03.06.2002
(51) Int. Cl.: A61F 2/36

(54) **Femurteil für eine Hüftgelenkendoprothese**
Femoral part for a hip joint endoprosthesis
Partie fémorale pour une endoprothèse de l'articulation de la hanche

(30) Priorität: 01.06.2001 DE 20109207 U
(43) Veröffentlichungstag der Anmeldung: 27.04.2005
(62) Teilanmeldung aus: 02730286.8
(73) Patentinhaber: Mathys AG Bettlach, 2544 Bettlach (CH); INSTITUT FÜR BIOPROZESS- UND ANALYSENMESSTECHNIK E.V., 37308 Heiligenstadt (DE)
(72) Erfinder: Glien, Wilfried, 07639 Bad Klosterlausnitz (DE); Liefeith, Klaus, 37318 Uder (DE); Rahm, Jens, 08412 Werdau (DE); Rost, Jürgen;c/o Inst. für Bioprozess- und, 37308 Heilbad Heiligenstadt (DE)
(74) Vertreter: Körfer, Thomas

(56) Entgegenhaltungen:
- EP-A- 0 528 284
- EP-A- 0 695 540
- EP-A- 0 808 618
- EP-A- 1 072 237
- WO-A-87/04916
- WO-A-96/04868
- DE-A- 3 829 361
- DE-C- 19 935 289
- DE-U- 9 290 043
- DE-U- 29 923 205
- FR-A- 2 662 932

## Beschreibung

Die Erfindung betrifft ein Femurteil für eine Hüftgelenkendoprothese, wobei das Femurteil einen Gelenkkopf, einen Hals und einen Schaft aufweist und der Schaft einen sich längs der Schaftachse verändernden Querschnitt aufweist und in der AP-Ebene (Anterior-Posterior-Ebene) um einen anterior gelegenen Punkt gekrümmt ist.

Aus DE-C1-199 35 289 ist eine Hüftgelenkendoprothese bekannt, bei der der konische Schaft in Längsrichtung verlaufende Nuten aufweist. Das Nutenprofil ist symmetrisch zum Mittelpunkt des Schaftquerschnitts und die Nuten verlaufen ohne Rotation über die gesamte Länge des Schaftes. Dieses Nutenprofil führt in Verbindung mit der Konizität des Schaftes zu einer Drehung des Trägheitsachsensystems längs der Schaftachse. Dadurch soll das Elastizitätsverhalten des Schaftes besser an die anatomischen Gegebenheiten des menschlichen Femurknochens angepasst werden, wodurch die Resorption des Knochens infolge Stress-Shielding verringert werden soll. Die in den Fig. 3a bis 3d dieser Druckschrift dargestellte Drehung des Trägheitsachsensystems beinhaltet eine Drehung der maximalen Hauptträgheitsachse von etwa +20^{•} gegenüber posterior.

Aus DE-A-38 29 361 ist eine einzementierbare Hüftgelenkendoprothese mit einem anatomisch geformten Schaft mit einem flanschartigen Stützkragen und mehreren Längsrillen bekannt. Die Längsrillen dienen der Bildung von Zementstegen und damit der Erhöhung der Rotations- und Torsionsfestigkeit und können nach der Zeichnung auch unsymmetrisch verlaufen.

Aus DE-T2-691 03 895 ist eine Hüftgelenkendoprothese bekannt, deren Schaft in seinem oberen Teil einen von unten nach oben zunehmend ovalen Querschnitt aufweist, wobei die Spitze nach innen und das andere Ende nach außen gerichtet ist, und eine Verwindung aufweist, die in einer in Richtung der Anteversion des Halses von unten nach oben zunehmenden Verdrehung besteht. Dadurch soll eine genaue Positionierung der Prothese und insbesondere eine Wiedergabe der Anteversion ermöglicht werden, die der Femurhals von Natur aus aufweist, und soll eine varale Versetzung des Femur verhindert werden.

Aus DE-U-92 90 043 ist eine anatomisch geformte Prothese aus Verbundmaterialien bekannt, deren Elastizitätsmodul dem des benachbarten kortikalen Knochens nach der Implantation angenähert ist.

Aus DE-U-299 23 205 ist ein einzementierbares Prothesenteil mit einem Kragen und einem anatomisch angepassten Schaft bekannt, wobei der proximale Schaftabschnitt eine Verwindung aufweist. Dadurch soll eine gleichmäßige, den anatomischen Verhältnissen angepasste Krafteinleitung erzielt werden.

Aus EP-A-0 695 540 ist eine Hüftgelenkendoprothese bekannt mit einer Drehung der Symmetrieebene der Schaftquerschnitte im oberen Bereich des Schaftes um etwa 10. Der Hals kann getrennt von dem Schaft hergestellt werden und mit seinem eigenen Anteversionswinkel an den Schaft angepasst sein, so dass dieser Anteversionswinkel mit dem Antetorsionswinkel des Schaftes zusammenwirkt.

Aus der EP-A-0 528 284 ist eine Hüftgelenkprothese für die Verankerung im Femur, mit einem blattartigen Schaft bekannt, wobei der Schaft sich von der Schulter zum unteren, distalen Ende hin verjüngt. Diese Hüftgelenkprothese umfasst einen zum Aufstecken eines Gelenkkopfs bestimmten, unter einem Winkel in den Schaft mündenden Hals, wobei der Schaft am an die Schulter anschließenden oberen, proximalen Bereich verwunden ist.

Bei diesen Femurteilen nach dem Stand der Technik dient die Krümmung des proximalen Bereichs der Anpassung an den Markraumkanal. Weiterhin soll dadurch dem Verkanten des Schaftes im Markraumkanal während der Implantation entgegengewirkt werden und eine gleichförmigere Stärke der Knochenzementschicht erzielt werden.

Die Problematik von Hüftendoprothesen ist der durch die Implantation einer Prothese sehr stark veränderte Kraftfluss im Oberschenkelknochen (Femur). Im intakten, prothetisch nicht versorgten System wird die Hüftreaktionskraft in den Femurkopf eingeleitet und über den relativ festen Rand (Kortikalis) des Femurs zum Knie abgeleitet. Die inneren Bereiche des Femurs (Markraum, Spongiosa) werden in dieser Konfiguration kaum belastet.

Die negativen Effekte einer implantierten Prothese auf den Femur lassen sich folgendermaßen formulieren:
Der überwiegende Teil der Hüftreaktionskraft wird über Prothesenkopf, -hals, sowie -schaft in das umgebende Knochengewebe geleitet. Ein Teil der Last wird bis zur Prothesenspitze geleitet und gelangt erst dort in den Femur. Das bedeutet eine starke Entlastung des periimplantären Knochens insbesondere im gesamten proximalen Bereich (stress-shielding). Da der Knochen dem natürlichen Gesetz der Effizienz unterworfen ist, resorbiert er entlastetes Knochengewebe (postoperatives Bone Remodeling). In der Folge verliert das Implantat sein stabiles Lager in der Knochenumgebung und lockert sich (aseptische Prothesenlockerung).

Eine weniger steife Prothese kann diesem Effekt in einem gewissen Maße insofern entgegenwirken, als das Implantat eine höhere Verformung zulässt und somit den umgebenden Knochen wesentlich weniger entlastet, d.h. stärker belastet, als eine steifere Prothese. Restriktionen an die konstruktive Gestaltung einer Prothese hinsichtlich ihrer Elastizität lassen diesen Überlegungen aber wenig Raum. So muss beispielsweise die Prothese die gleiche Festigkeit aufbringen, wie zuvor der gesunde Knochen um die Hüftreaktionskraft zu tragen, mit der Folge, dass sich nun im ursprünglich weichen und kaum belasteten Markraum und Spongiosabereich des Knochen ein steifes lasttragendes Implantat befindet.

Bei zementierten Prothesensystem kommt das Problem der Belastung des Knochenzements hinzu. Der Knochenzement (ein PMMA) reagiert empfindlich auf Wechselbelastungen, die um so höher sind, je elastischer das Implantat ausgelegt ist. Die Langzeitstabilität des Zement wird also von der Amplitude der Belastung, der Anzahl der Schwingungen [(1-3 Mill.)/Jahr] und zusätzlich von Alterungserscheinungen des Zementes selbst beeinflusst. Um eine lange Standzeit des Implantats zu gewährleisten ist somit der Knochenzement möglichst zu schonen. Daraus folgt, dass eine gewünschte Mehrbelastung im Femur, also ein verringertes Stress-Shielding, in der Regel zu einer unerwünschten Mehrbelastung des Knochenzements führt. Die Belastungen des postoperativen Femurs ist also einerseits erwünscht, um einer aseptischen Prothesenlockerung vorzubeugen, führt anderseits leicht zu einer übermäßigen Beanspruchung des Knochenzements.

Der Erfindung liegt die Aufgabe zugrunde, das Lockern des implantierten Hüftgelenkschafts zu verzögern.
Die Aufgabe wird durch die Merkmale des Anspruchs 1 gelöst.

Erfindungsgemäß wird diese Aufgabe u.s. dadurch gelöst, dass zumindest in einem Längsabschnitt des Femurteils die maximale Hauptträgheitsachse nicht posterior ausgerichtet ist.

Erfindungsgemäß beträgt die Abweichung von der posterioren Ausrichtung ± 10 bis 30°.

Die Änderung des Schaftquerschnitts ist derart, daß sich die Richtung seiner Hauptträgheitsachsen längs des Schaftes dreht. Der Prothesenschaft hat eine sich entlang der Prothesenlängsachse verändernde Richtung der Hauptachsen der Flächenträgheitssysteme. Die Änderung ist durch die Festlegung von Winkeln zwischen einem festen Bezugsachsensystem und den Hauptachsen der Flächenträgheitssysteme definiert. Diese Winkel können sowohl negativ als auch positiv sein und liegen vorzugsweise zwischen 10 und 30°.

Die Ausrichtung der Hauptträgheitsachsen des Femurs ändert sich längs der Achse des Femurs. Die Änderung der Ausrichtung der Hauptträgheitsachsen des erfindungsgemäßen Endoprothesen-Femurteils kann dem zumindest teilweise angepasst werden. Welche Lösung im Einzelfall gewählt wird, muss der Operateur entscheiden. Durch die Erfindung hat er eine größere Auswahlmöglichkeit, um den im Einzelfall optimalen Kompromiss zwischen Schonung des Knochenzements und Verringerung des Stress-Shielding zu finden. Das Stress-Shielding lässt sich dabei auch durch die Materialauswahl steuern. Ein relativ weiches Material, z.B. eine Titanlegierung, verringert das Stress-Shielding stärker als ein steifes Material, z.B. eine Kobalt-Chrom-Legierung.

Zur Erklärung des durch die Erfindung erzielten Effektes werden nachfolgend die Systeme der Hauptträgheitsachsen des natürlichen Femur und des Implantats betrachtet:

Hauptträgheitsachsen spielen als mechanische Größe eine Rolle, wenn Biegung auftritt. Ein Hauptträgheitsachsensystem weist immer eine maximale und eine minimale Trägheitsachse auf. Diese charakterisieren jene Achsen, welche bei einer Biegung der Querschnittsfläche um diese Achsen ein maximales Widerstandsmoment (maximale Hauptträgheitsachse) bzw. ein minimales Widerstandsmoment (minimale Hauptträgheitsachse) durch diese Fläche hervorrufen. Der Femur wird wie die meisten Knochen u.a. auf Biegung beansprucht und im Femur existiert ein System von Hauptträgheitsachsen. Für jeden Femurquerschnitt existiert also eine maximale und eine minimale Hauptträgheitsachse.

Überlagert man alle Belastungen, denen der Femur ausgesetzt ist (Treppensteigen, Gehen, Laufen, Springen usw.), zu einer einzigen, mehrachsigen Belastung, so bestimmt die Biegebelastung des Femur im Wesentlichen die Orientierungen der Hauptachsensysteme.

Bei der Implantation eines Prothesenschaftes überlagert das System von Trägheitsachsen der Querschnitte des Implantats das vorgegebene System des Femur. Es stellen sich im Verbundsystem Femur/Implantat neue Hauptachsensysteme ein. Abgesehen von der eintretenden Versteifung, der Verringerung der Biegebelastung des periimplantären Knochengewebes und dem veränderten Kraftfluss, mit teilweisem oder völligem Verlust der Belastung in bestimmten Bereichen des Femur, bestehen zwei Extremsituationen hinsichtlich der Hauptachsensysteme:

1. Die maximale Hauptträgheitsachse der Prothese ist so gerichtet wie die maximale Hauptträgheitsachse des Femur (querschnittsweise betrachtet). Das bedeutet, die ursprünglich bestehenden lokalen steifen und weniger steifen Regionen im Femur bleiben auch im Verbundsystem erhalten. Die Belastungsverteilung ist also ähnlich wie im Femur ohne Implantat und das resultierende Stress-Shielding des Knochengewebes ist verringert.

Für ein einzementiertes Implantat ist das Vorhandensein von Regionen unterschiedlicher Belastung jedoch ungünstig. Die Zementbelastung ist immer dann am geringsten, wenn die Belastung möglichst homogen in den Zement eingeleitet wird. Existieren aber nun unterschiedliche Steifigkeiten ist das nicht möglich. In den Bereichen geringer Steifigkeit ist die Zementbelastung am höchsten.

2. Die maximale Hauptträgheitsachse der Prothese ist so gerichtet wie die minimale Hauptträgheitsachse des Femur. Die ursprünglich bestehenden steifen und schwachen Regionen des Femur werden verändert und die schwachen Bereiche des Femur werden durch das Implantat versteift und umgekehrt. Dies bedeutet ein erhöhtes Stress-Shielding. Anderseits wird der Unterschied zwischen maximalen und minimalen Widerstandsmomenten der Fläche kleiner, was eine homogenere Lasteinleitung in den Knochenzement bedeutet.

Überlagert wird das Ganze natürlich sehr stark von der Entlastung des Femur durch die Prothese selbst (Stress-Shielding) und durch andere Belastungen des Femur wie Torsion und Druckbelastung (Knochenzement).

Unter Berücksichtigung der konstruktiven Vorgaben für den Femurschaft einer Hüftgelenkendoprothese ergibt sich Folgendes: Am oberen Ende des Schaftes ist die maximale Hauptträgheitsachse häufig konstruktionsbedingt posterior gerichtet. Wird bei einem zementierten Prothesenschaft in erster Linie eine Schonung des Knochenzements angestrebt, so kann sich die Ausrichtung der maximalen Haupträgheitsachse bspw. entsprechend einer Sinusfunktion zwischen 0 und n/2 längs der Schaftachse um bis zu 30° zu einer posterior-medialen Ausrichtung ändern. Die Richtung der minimalen Hauptträgheitsachse des Schaftquerschnittes hat dann im distalen Bereich etwa die Richtung der maximalen Hauptträgheitsachse des Femurs. Mit einem solchen Schaft lässt sich die Zementbelastung besonders stark verringern. Das Stress-Shielding ist dagegen nur wenig reduziert.

Soll als Vergleichsbeispiel bei einem zementierten Prothesenschaft dagegen in erster Linie das Stress-Shielding verringert werden, so kann ein Femurteil eingesetzt werden, bei dem die maximale Hauptträgheitsachse am oberen Ende posterior gerichtet ist und sich entsprechend einer Sinusfunktion zwischen 0 und π/2 längs der Schaftachse und bis zu 30° in posterior-laterale Richtung dreht.

Bei einer solchen zementierten Prothese wird durch das optimierte Prothesensystem eine bisher nicht mögliche Kombination von geringerer Entlastung des postoperativen Femurs und geringerer Belastung des Knochenzements erreicht. D.h. das infolge der Implantation auftretende Stress-Shielding wird nur so weit reduziert, dass nicht unvertretbar hohe Zementbelastungen auftreten.

Bei einem unzementierten Prothesenschaft mit einer posterioren Ausrichtung der maximalen Hauptträgheitsachse am proximalen Ende des Schaftes, lässt sich in Vergleich zur Erfindung die Lebensdauer des Implantats dadurch verlängern, dass die Richtungen der maximalen Hauptträgheitsachse des Femurteils der Prothese und des Femur möglichst weit übereinstimmen. Bei einem unzementierten Prothesenschaft braucht keine Zementbelastung berücksichtigt werden, so daß ein größerer Spielraum für den Verlauf der Hauptträgheitsachsen längs des Prothesenschafts besteht. Wird dabei eine höhere Belastung des Femurs beabsichtigt, so kann sich die maximale Hauptträgheitsachse längs des Prothesenschaftes entsprechend einer Sinusfunktion zwischen 0 und n/2 in die posterior-laterale Richtung drehen. Bei einer unzementierten Prothese wird dadurch eine geringere Entlastung des postoperativen Femurs erreicht. D.h. das infolge der Implantation auftretende Stress-Shielding wird reduziert.

Die maximalen Hauptträgheitsachsen von Querschnitten des Femurs zeigen in der Resektionsebene überwiegend in lateral-posteriore Richtung. Um bereits am proximalen Ende des Schaftes eine Annäherung der Ausrichtung dessen maximaler Hauptträgheitsachse an die des Femur zu erzielen, werden anterior und/oder posterior Rillen oder ähnliche bei nicht erfindungsgemäßen Prothesen Aussparungen in der Oberfläche des Schaftes vorgesehen. Um die maximale Hauptträgheitsachse entsprechend denen des Femurs posterior-lateral auszurichten, sind im posterior-lateralen Bereich und/oder im anterior-medialen Bereich der Schaftoberfläche jeweils eine Rille vorgesehen.

Das gleiche Ergebnis wird erfindungsgemäß durch einen schalenförmigen Aufbau des Schaftes mit gedrehtem Kern erreicht, wobei für die Schale und den Kern unterschiedliche Materialien verwendet werden und der Kern auch leer sein kann.

Ist auf diese Weise die Ausrichtung der maximalen Hauptträgheitsachse bereits in der Resektionsebene um 10 bis 30° nach lateral oder medial gedreht, so kann diese Ausrichtung auch über die gesamte Länge des Femurteils beibehalten werden. In diesem Fall dreht sich die Ausrichtung der maximalen Hauptträgheitsachse dann nicht entlang des Femurteils.

Vorzugsweise ist der Schaft in der AP-Ebene zweifach gekrümmt (S-Krümmung), wobei der proximale Schaftbereich einen anterior gelegenen Krümmungsmittelpunkt und einen Krümmungsradius im Bereich von 250 mm ±100 mm hat und der distale Schaftbereich einen posterior gelegenen Krümmungsmittelpunkt und einen Krümmungsradius im Bereich von >250mm, insbesondere 350 mm ±100 mm. Die Krümmung des distalen Bereichs ist somit in Regel kleiner als die des proximalen Bereichs und kann auch ganz entfallen. Es handelt sich hierbei nicht um durchgängig konstante Krümmungen, sondern die Krümmungen variieren über diese Bereiche. Die variable Spanne überdeckt sowohl die anatomisch unterschiedlichen Femora als auch die unterschiedlichen Krümmungsradien in dem proximalen und dem distalen Bereich und die unterschiedlichen Prothesengrößen.

Bei der Erfindung besitzt der Prothesenschaft eine S-Krümmung und gleichzeitig liegen die maximalen Hauptträgheitsachsen der Schaftquerschnitte nicht in einer Ebene. Diese Ausführungsform des erfindungsgemäßen Femurteils wird insbesondere für die zementierte Implantation verwendet. Durch die S-Krümmung ergibt sich eine genauere Anpassung des Prothesenschafts an die natürliche Kurvatur des Femur, wodurch die einzelnen Bereiche des Zementes eine gleichförmigere Dicke haben und dadurch gleichmäßiger belastet werden. Dadurch wird die Möglichkeit eröffnet, auch bei Anpassung der maximalen Hauptträgheitsachsen der Schaftquerschnitte längs der Schaftachse an die maximalen Hauptträgheitsachsen der Femurquerschnitte (Verringerung des Stress-Shielding) die Belastung auf den Zementköcher weitgehend gleichmäßig einzustellen.

Mittels dieser Ausführungsform gelingt es also, den oben erwähnten Gegensatz zwischen Verringerung der Zementbelastung und Verringerung des Stress-Shielding weiter abzubauen und den Knochenzement gering zu belasten, den Knochen selbst dagegen möglichst hoch, d.h. möglichst unverändert gegenüber der preoperativen Situation. Dieses "unverändert" betrifft dabei nicht nur die Stärke der Knochenbelastung, sondern auch die Art und Weise seiner Belastung, und zwar die möglichst gute Übereinstimmung der Biegeeigenschaften des Femur pre- und postoperativ.

Bei den bekannten Prothesen wird man bei der Einleitung der Hüftgelenkskraft stark belastete Bereiche und gering belastete Bereiche im Zement finden. Die stark belasteten Bereiche stellen nun aber die Gefahr für die Integrität des Zementköchers dar, weil diese Spannungsspitzen in Verbindung mit der auftretenden Dauerschwingbelastung den Zement schädigen und zum Versagen lokaler Gebiete im Zement führen, was wiederum zum Lockern der Prothese führen kann.

Da die aseptische Lockerung des Implantats sowohl von der Zementzerrüttung als auch der Knochenresorption verursacht wird, bringt die alleinige Verringerung der Zementbelastung oder die Erhöhung der Femurbelastung nur eine geringe Verlängerung der Standzeit. Die Prothese kann sich nämlich immer noch infolge der jeweils anderen nicht verbesserten Ausfallursache lockern. Die Erhöhung der Femurbelastung und die Verringerung der Zemententlastung führen jedoch in Verbindung miteinander zu einer wesentlich gesteigerten Standzeit des Implantats.

Vorzugsweise ist das erfindungsgemäße Femurteil bei einer zementfreien Implantation aus einer Titanlegierung und bei einer zementierten Implantation aus einem anderen biokompatiblen Material hergestellt, z.B. einer CoCrMo-Legierung.

Ein Ausführungsbeispiel der Erfindung wird nachfolgend anhand der Zeichnung erläutert. Es zeigen:
- Fig. 1: das nicht erfindungsgemäße Femurteil einer rechten Hüftgelenkendoprothese für zementierte oder zementfreie Implantation;
- Fig. 2: das Femurteil einer erfindungsgemäßen Hüftgelenkendoprothese für eine zementierte Implantation;
- Fig. 3: einen Querschnitt des rechten Femurs mit implantierter Prothese und dem jeweiligen Hauptachsensystem;
- Fig. 4: die Trägheitshauptachsensysteme von Querschnitten eines rechten Femurschafts mit sich ändernder Richtung der Hauptträgheitsachsen;
- Fig. 5: eine Ansicht des Femurschafts von posterior;
- Fig. 6: eine Ansicht des Femurschafts von anterior;
- Fig. 7: einen Schnitt des Femurschafts nach 7-7 von Fig. 5;
- Fig. 8: einen Schnitt des Femurschafts nach 8-8 von Fig. 5;
- Fig. 9: einen Femurschaft im Teil-Längsschnitt;
- Fig. 10: den Femurschaft von Fig. 9 im Schnitt nach 10-10 und
- Fig. 11: den Femurschaft von Fig. 9 im Schnitt nach 11-11.

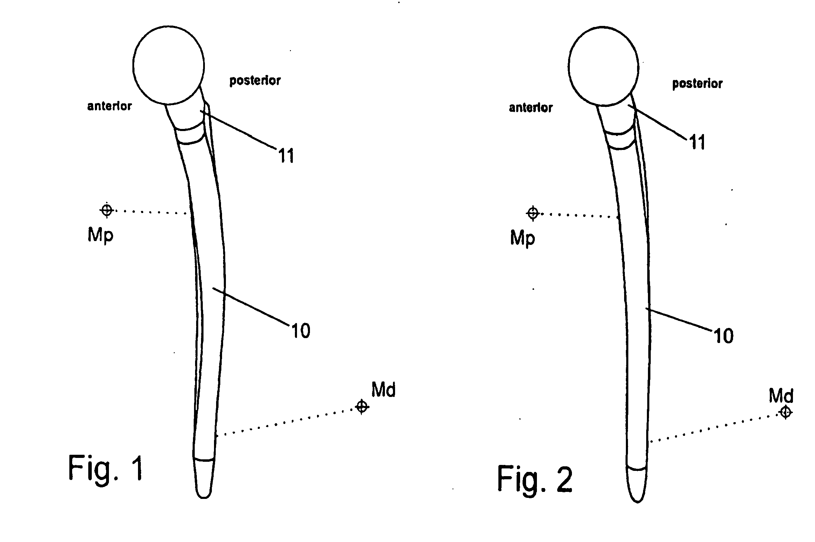

Fig. 1 und 2 zeigen das Femurteil einer Hüftgelenkendoprothese, und zwar für das rechte Hüftgelenk. Das Femurteil besteht aus einem Schaft 10, der an seinem oberen Ende auf einem Hals 11 einen Gelenkkopf 12 trägt.

Der Schaft 10 ist in der AP-Ebene S-förmig gekrümmt. Der proximale Schaftbereich hat einen anterior gelegenen Krümmungsmittelpunkt Mp, während der distale Schaftbereich gerade ist oder einen posterior gelegenen Krümmungsmittelpunkt Md hat. Die Krümmung ist nicht gleichförmig und ist im distalen Bereich kleiner als im proximalen Bereich. Im proximalen Bereich liegt der Krümmungsradius zwischen 150 und 350 mm und im distalen Bereich zwischen 250 mm und m. Typische Werte sind 250 bzw. 350 mm.

Der Zweck der Schaftkrümmung in der AP-Ebene ist eine bessere Anpassung des Implantates an den gekrümmten Verlauf des Markraumkanals. Weiterhin wird dem Verkanten des Schaftes im Markraumkanal während der Implantation entgegengewirkt und eine homogenere Verteilung des Knochenzementes erzielt.

Fig. 1 zeigt ein nicht erfindungsgemäßes rechtes Femurteil, bei dem die maximale Hauptträgheitsachse am proximalen Ende posterior gerichtet ist und sich längs der Schaftachse nach posterior-lateral dreht.

Ein solches Femurteil kann bei zementfreien und zementierten Prothesensystemen eingesetzt werden, wenn die Verringerung des Stress-Shielding im Vordergrund steht. Bei einem zementierten Prothesensystem ist dabei die Zementbelastung relativ hoch.

Fig. 2 zeigt ein erfindungsgemäßes rechtes Femurteil, bei dem die maximale Hauptträgheitsachse des Schaftes am proximalen Ende ebenfalls posterior gerichtet ist, sich jedoch längs der Schaftachse nach posterior-medial dreht. Ein solches Femurteil kann bei einem zementierten Prothesensystem eingesetzt werden, wenn auf eine Entlastung des Zements Wert gelegt wird und weniger Wert auf eine Verringerung des Stress-Shielding.

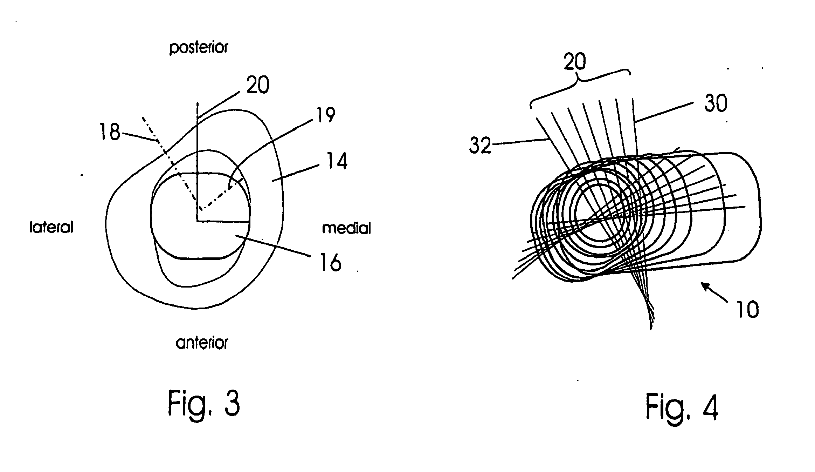

Fig. 3 zeigt im Schnitt einen rechten Femur 14 mit eingesetztem herkömmlichem Implantat 16. Untersuchungen an Femora haben gezeigt, dass die maximale Hauptträgheitsachse 18 überwiegend in lateral-posteriore Richtung weist. Die maximale Hauptträgheitsachse 20 der Schaftprothese des herkömmlichen Implantats 16 weist dagegen in posteriore Richtung. Diese Diskrepanz zwischen der maximalen Hauptträgheitsachse des Femurteils der Prothese und der minimalen Hauptträgheitsachse des Femurs führt bei zementierten Implantaten zu einer ungünstigen Belastung des Knochenzements. Es hat sich gezeigt, dass eine Verringerung des Winkels zwischen der maximalen Hauptträgheitsachse 20 des Implantats 16 und der minimalen Hauptträgheitsachse 19 des Femurs die Belastung des Knochenzements verringert.

Bei dem erfindungsgemäßen Femurteil haben die Schaftquerschnitte über die Länge des Schafts 10 Hauptträgheitsachsen 20, die nicht parallel zueinander liegen, sondern in ihrer Richtung voneinander abweichen. Die Abweichung ist dabei derart, dass die maximalen Hauptträgheitsachsen 20 des Implantats nicht in der AP-Ebene liegen, sondern unter einem Winkel zueinander verlaufen.

Das richtungsveränderliche Hauptachsensystem des Prothesenschafts 10 weist eine Drehung der Schaftquerschnitte um die Schaftachse auf. Am proximalen Ende (Resektionsebene) ist die maximale Hauptträgheitsachse z.B. posterior gerichtet, so dass sie insoweit mit einem herkömmlichem Implantat 16 übereinstimmt. Längs der Schaftachse zum distalen Ende hin dreht sich die maximale Hauptträgheitsachse beim erfindungsgemäßen Femurteil um bis zu etwa 30°. Die Drehung nimmt dabei entsprechend etwa einer Sinusfunktion im Bereich von 0 bis n/2 zu.

Steht bei einzementierten Prothesenschäften die Entlastung des Knochenzements im Vordergrund, so kann sich die Richtung der maximalen Hauptträgheitsachse 20 des Prothesenschafts 10 entsprechend der erwähnten Sinusfunktion der Richtung der minimalen Hauptträgheitsachse des Femurquerschnitts anpassen. Dies bedeutet, dass sich die Richtung der maximalen Hauptträgheitsachse von der z.B. posterioren Ausrichtung am proximalen Ende zur posterior-medialen Ausrichtung am distalen Ende dreht. Beim rechten Prothesenschaft entspricht dies der Drehung eines rechtsgängigen Gewindes und beim linken Prothesenschaft der Drehung eines linksgängigen Gewindes.

Bei einer nicht erfindungsgemäßen zementfrei implantierten Prothese sollen im Gegensatz dazu die Wechselwirkungskräfte zwischen dem Femur und dem Prothesenschaft erhöht werden, um die ansonsten stattfindende Resorption des entlastenden Knochengewebes zu vermeiden (Stress-Shielding). Auch bei zementierten Prothesenschäften wird bisweilen mehr Wert auf eine Verringerung des stress-shielding als auf eine Entlastung des Zements gelegt. Die Richtung der maximalen Hauptträgheitsachse zeigt dann am proximalen Ende des Prothesenschafts 10 z.B. konstruktionsbedingt wieder in posteriore Richtung. Zum distalen Ende hin dreht sie sich entsprechend einer Sinusfunktion im Bereich von 0 bis n/2 um bis zu 30° in laterale Richtung. Die Drehung ist daher beim rechten Prothesenschaft linksgängig und beim linken Prothesenschaft rechtsgängig.

Fig. 4 zeigt - von oben betrachtet - die Richtung der beiden Hauptträgheitsachsen für Querschnitte an verschiedenen Punkten längs des Prothesenschafts 10. Zur Vereinfachung der Darstellung ist der Prothesenschaft 10 dabei als gerade angenommen, d.h. die Krümmung in der AP-Ebene ist nicht dargestellt. Am proximalen Ende ist die maximale Hauptträgheitsachse entsprechend der Linie 30 ausgerichtet. Diese Ausrichtung dreht sich zum distalen Ende hin dann zu der Linie 32.

In Fig. 4 hat der Prothesenschaft 10 einen mehr oder weniger stark ausgeprägten ovalen Querschnitt, der jeweils zu den Hauptträgheitsachsen symmetrisch ist, wobei der Prothesenschaft im Inneren homogen ist.

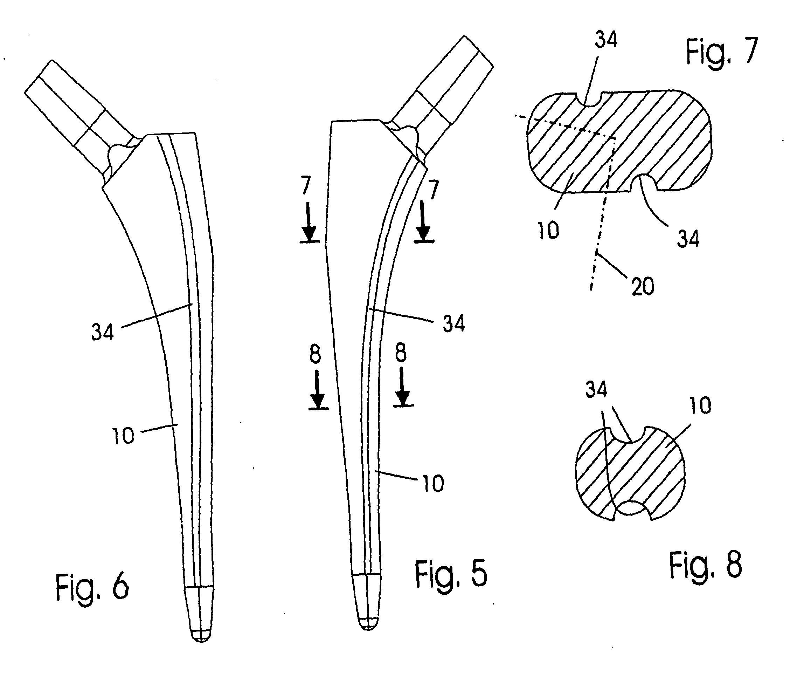

Bei den Ausführungsbeispielen der Figuren 1 bis 4 ist die Oberfläche des Femurteils glatt, d.h. etwa oval und frei von einspringenden und vorstehenden Konturen, und die Drehung des Hauptträgheitsachsensystems wird alleine durch Drehung der Querschnitte längs des Prothesenschafts erreicht. Wie insbesondere bei zementierten Implantaten üblich, kann die Oberfläche jedoch porös sein, um das Einwachsen des Knochengewebes zu fördern. Die Oberfläche muss jedoch bei nicht erfindungsgemäßen Prothesen nicht glatt sein und die Richtung der Hauptträgheitsachsen kann auch durch Veränderungen der äußeren Kontur des Prothesenquerschnitts, z.B. das Einbringen von Radien, Rillen oder Kerben erreicht werden, die zu einer Drehung der Hauptträgheitsachsen entlang des Prothesenschafts 10 führen. Fig. 5 bis 8 zeigen einen Femurschaft, der durch asymmetrisch posterior und anterior auf der Oberfläche des Femurschafts vorgesehene Rillen 34 einen Querschnitt hat, dessen Hauptträgheitsachsen sich längs des Femurschafts rechtsgängig drehen. Die Rillen 34 verändern den Querschnitt des Schaftes 10 dabei so, dass bereits in der Resektionsebene die maximale Hauptträgheitsachse um einige Grad in die posterior-laterale Richtung gedreht ist und damit bereits hier ungefähr entsprechend der maximalen Hauptträgheitsachse des Femurs ausgerichtet ist. Entlang der Schaftachse dreht sich die maximale Hauptträgheitsachse des Schaftes 10 dann entsprechend der des Femurs, wobei der Drehungswinkel hier kleiner ist, da die maximale Hauptträgheitsachse am proximalen Ende bereits weitgehend mit der des Femurs ausgerichtet ist.

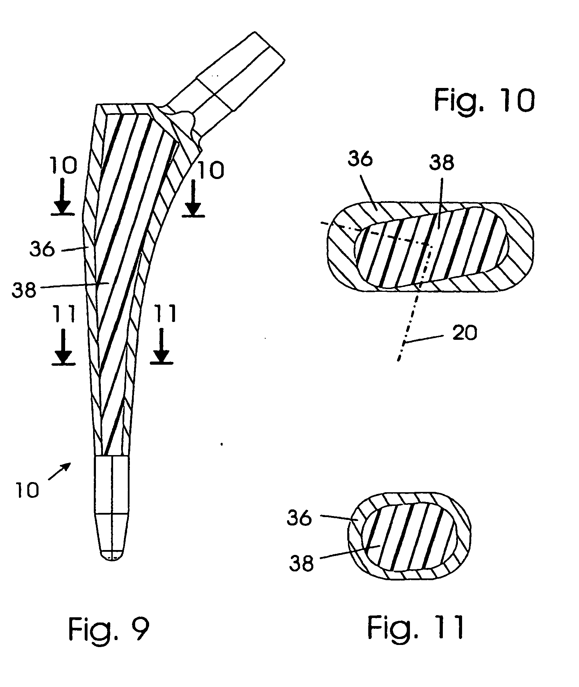

Das gleiche lässt sich auch durch Abweichungen vom homogenen Querschnitt, also durch erfindungsgemäße Veränderungen der inneren Gestalt des Prothesenschafts 10 erreichen, z.B. durch das Einbringen von Hohlräumen, Bohrungen, Gussformen, Rillen, Kerben u.dgl. Solche vorab eingebrachten Hohlräume können mit Materialien anderer Elastizität und Steifigkeit ausgefüllt werden. Fig. 9, 10 und 11 zeigen ein solches Ausführungsbeispiel für ein linkes Femurteil, bei dem die oberflächennahen Bereiche 36 des Prothesenschafts 10 aus einem Material mit besonders hoher mechanischer Festigkeit besteht, während der innere Bereich 38 aus weicherem Material besteht und sogar ein Hohlraum sein kann. Wie ein Vergleich der Fig. 10 und 11 zeigt, ist die Wandstärke am distalen Ende des Prothesenschafts dabei etwa gleichförmig, während sie am proximalen Ende auf dem Umfang unterschiedlich ist. Der Querschnitt des Prothesenschafts ist am proximalen Ende stark oval. Auch der innere Bereich 38 hat einen ovalen Umriss, der jedoch gegenüber dem äußeren Umriss des Prothesenschafts 10 gedreht ist, so dass die maximale Hauptträgheitsachse in der Resektionsebene aus posteriorer Richtung um einige Grad in laterale Richtung gedreht ist.

Schließlich kann eine Drehung des Hauptträgheitsachsensystems auch noch durch die Verwendung von anisotropen, faser- oder partikelverstärkten Werkstoffen erreicht werden.

## Patentansprüche

1. Femurteil für eine Hüftgelenkprothese mit einem Schaft (10), einem Hals (11) und einem Gelenkkopf (12),
wobei der Schaft (10) einen sich längs der Schaftachse verändernden Querschnitt aufweist,
wobei der proximale Bereich des Schaftes (10) in der AP-Ebene um einen anterior gelegenen Punkt (Mp) gekrümmt ist,
wobei der distale Schaftbereich in der AP-Ebene um einen posterior gelegenen Mittelpunkt (Md) gekrümmt ist;
wobei die maximale Hauptträgheitsachse des Schaftquerschnitts am proximalen Ende (Resektionsebene) etwa posterior ausgerichtet ist und sich zum distalen Ende hin um etwa 10 bis 30° um die Schaftachse in posterior-mediale Richtung dreht,
und wobei die Oberfläche von einspringenden oder vorstehenden Konturen frei ist,
**dadurch gekennzeichnet,**
**dass** die Hauptträgheitsachsen der Querschnittsflächen über die Schaftlängsachse durch geometrische oder konstruktive Veränderungen der inneren Struktur des Prothesenquerschnittes verdreht sind.

2. Femurteil nach Anspruch 1,
**dadurch gekennzeichnet,**
**dass** die Veränderung der inneren Struktur durch das Einbringen von Hohlräumen, die Verwendung von anisotropen, faser- oder partikelverstärkten Werkstoffen oder das Ausfüllen vorab eingebrachter Hohlräume mit Materialien anderer Elastizität und Steifigkeit erfolgt.

3. Femurteil nach Anspruch 2,
**dadurch gekennzeichnet,**
**dass** die Hohlräume durch Bohrungen, Gussformen, Rillen oder Kerben gebildet sind.

4. Femurteil nach einem der Ansprüche 1 bis 3,
**dadurch gekennzeichnet,**
**dass** der Krümmungsradius des proximalen Schaftbereichs kleiner ist als der des distalen Schaftbereichs.

5. Femurteil nach Anspruch 4,
**dadurch gekennzeichnet,**
**dass** der Krümmungsradius des proximalen Schaftbereichs etwa 250 mm ±100 mm und der des distalen Schaftbereichs etwa 350 ±100 mm beträgt.

## Claims

1. A femur part for a hip joint prosthesis with a stem (10), a neck (11) and a joint head (12),
the stem (10) exhibiting a cross-section which varies along the stem axis,
the proximal area of the stem (10) being curved in the AP plane about a point (Mp) in anterior position,
the distal stem area being curved in the AP plane about a mid-point (Md) in posterior position
the maximum principal axis of inertia of the stem cross-section at the proximal end being of approximately posterior orientation and turning towards the distal end by around 10 to 30° about the stem axis in the posterior-medial direction,
and the surface being free from re-entrant or projecting contours,
**characterised in that**
the principal axes of inertia of the cross-sectional surfaces are twisted over the longitudinal axis of the stem by geometric or structural changes to the inner structure of the prosthesis cross-section.

2. A femur part according to claim 1, **characterised in that** change to the inner structure is brought about by the introduction of cavities, the use of anisotropic, fibre- or particle-reinforced materials or the filling of cavities introduced beforehand with materials of different elasticity and rigidity.

3. A femur part according to claim 2, **characterised in that** the cavities are formed by drill holes, moulds, grooves or notches.

4. A femur part according to one of claims 1 to 3, **characterised in that** the radius of curvature of the proximal stem area is smaller than that of the distal stem area.

5. A femur part according to claim 4, **characterised in that** the radius of curvature of the proximal stem area amounts to approximately 250 mm ± 100 mm and that of the distal stem area amounts to approximately 350 ± 100 mm.

## Revendications

1. Partie fémorale pour prothèse de la hanche avec une tige (10), un col (11) et une tête d'articulation (12), la tige (10) ayant une section qui change le long do l'axe de la tige,
la région proximale de la tige (10) étant recourbée dans le plan AP autour d'un point situé côté antérieur (Mp),
la région distale de la tige étant recourbée dans le plan AP autour d'un point central situé côté postérieur (Md),
l'axe d'inertie maximal principal de la section de la tige étant dirigé dans la direction approximativement postérieure au niveau de l'extrémité proximale et tournant autour de l'axe de la tige dans le sens postéro-latéral d'environ 10 à 30° lorsqu'on s'approche de l'extrémité distale,
et la surface étant exempte de contours en creux ou en saillie,
**caractérisée en ce que**
les axes d'inertie principaux des surfaces de section sont toronnés sur l'axe longitudinal de la tige par des modifications de géométrie ou de construction de la structure interne de la section de la prothèse.

2. Partie fémorale selon la revendication 1,
**caractérisée en ce que**
la modification de la structure interne a lieu par l'insertion de creux, l'utilisation de matières anisotropes renforcées par des fibres ou des particules ou le remplissage de creux préalablement insérés avec des matières ayant une autre élasticité et rigidité.

3. Partie fémorale selon la revendication 2,
**caractérisée en ce que**
les creux sont formés par des alésages, des formes moulées, des rainures ou des encoches.

4. Partie fémorale selon l'une quelconque des revendications 1 à 3,
**caractérisée en ce que**
le rayon de courbure de la région proximale de la tige est inférieur à celui de la région distale de la tige.

5. Partie fémorale selon la revendication 4,
**caractérisée en ce que**
le rayon de courbure de la région proximale de la tige est d'environ 250 mm ± 100 mm et celui de la région distale de la tige d'environ 350 ± 100 mm.
